# EUROPEAN PATENT SPECIFICATION

(11) **EP 4 006 165 B1**
(45) Date of publication and mention of the grant of the patent: **29.04.2026**
(21) Application number: 20847306.6
(22) Date of filing: 21.07.2020
(51) Int. Cl.: C12Q 1/6851

(54) **MULTIPLEX LIGATION-DEPENDENT PROBE MICROARRAY DETECTION**
MULTIPLEX-LIGATIONSABHÄNGIGE SONDENMIKROARRAY-DETEKTION
DÉTECTION DE MICRORÉSEAU DE SONDES DÉPENDANT DE LA LIGATURE MULTIPLEX

(30) Priority: 31.07.2019 CN 201910701155
(43) Date of publication of application: 01.06.2022
(73) Proprietor: Flashdx Shenzen Inc., Guangdong 518057 (CN)
(72) Inventor: YUE, Min, Shenzheng, Guangdong 518057 (CN); DAI, Heng, Shenzheng, Guangdong 518057 (CN)
(74) Representative: dompatent
(86) International application number: PCT/CN2020/103336
(87) International publication number: WO 2021/017955

(56) References cited:
- WO-A2-03/054511
- CN-A- 104 357 549
- CN-A- 108 220 399
- CN-A- 110 016 500
- CN-A- 110 055 304
- CN-A- 111 621 551
- US-A1- 2004 110 134
- US-A1- 2017 362 648

## Description

### Technical field

The present invention relates to the field of nucleic acid detection, more particularly to the detection of multiplex ligation probe microarrays.

### Background

In various fields such as medicine, food, hygiene, etc., nucleic acid detection is often required on the sample to be tested. At present, commonly used detection methods include conventional PCR, quantitative PCR, probe detection, chip detection, sequencing and other technologies.

In conventional real-time fluorescent quantitative (qPCR), primer pairs for amplification and probes for detection are present in the detection system. Among them, one side (or one end) of the probe in the solution has a fluorescent agent (fluorophore), and the other side (or the other end) has a quencher. When the probe is intact, because the quencher and the fluorophore are close enough, the quencher can effectively inhibit the fluorophore, so that there is no (or substantially no) fluorescent signal. When there is a nucleic acid sample matching the sequence of the probe in the solution, the probe is cleaved by the amplification enzyme during the PCR amplification of the sample, so that the fluorophore and the quencher will be separated from each other, resulting in a reduction or disappearance of the inhibitory effect, thereby allowing the fluorophore to generate a fluorescent signal.

However, the current real-time fluorescent quantitative PCR still has some shortcomings, including: because the probe is labeled with a fluorophore and a quencher at the same time, the cost of probe synthesis is high; in order to make the probe in the solution cleaved by the amplification enzyme, amplification enzyme needs to have 5'→3' exonuclease activity, however some amplification enzymes do not have 5'→3' exonuclease activity and are therefore not suitable for qPCR. More importantly, because each sequence to be tested requires a fluorophore whose emission spectrum can be distinguished from other fluorophores, the number of different nucleic acids that can be detected simultaneously in the same qPCR reaction is greatly limited, usually a qPCR reaction can only achieve 4-5 multiplex detection. In addition, even if multiplex qPCR can be realized, because multiple different fluorescent agents need to be read at the same time, the corresponding optical detection equipment is complicated in design and expensive.

Therefore, there is an urgent need in the art to develop a method for quantitative detection of specific sequences by using only one pair of primers and multiple pairs of probes to perform multiplex ligation-dependent amplification.

US 2004/110134 A relates to methods and kits for quantitating target nucleic acid sequences using coupled ligation and amplification. It also relates to methods, reagents, and kits that employ addressable-support specific portions.

WO 03/054511 A2 relates to methods and kits for quantitating target nucleic acid sequences using coupled ligation and amplification, as well as to methods, reagents, and kits that employ addressable-support specific portions.

US 2017/362648 A1 provides methods and systems for measuring the concentration of multiple nucleic acid sequences in a sample. The nucleic acid sequences in the sample are simultaneously amplified, for example, using polymerase chain reaction (PCR) in the presence of an array of nucleic acid probes.

CN 110 016 500 A provides a quantitative PCR method based on a surface probe as well as a corresponding detection method, a detection device and an application.

### Summary of the invention

The purpose of the present invention is to provide a quantitative detection method for a specific sequence by using only one pair of primers and multiple pairs of probes to perform multiplex ligation-dependent amplification.

The invention is set out in the appended set of claims.

In a first aspect of the present invention, it provides a quantitative PCR detection system based on a surface probe, and the detection system comprises:
(a) a solid phase carrier, one primary surface of the solid phase carrier is provided with n sub-detection regions, wherein n is a positive integer ≥ 2, and at least one sub-detection region is a surface quantitative sub-detection region;
   wherein, each of the surface quantitation sub-detection region is independently immobilized with a quenching product capturing nucleic acid, the quenching product capturing nucleic acid is a single-stranded nucleic acid, and one end of the quenching product capturing nucleic acid is immobilized on the surface of the solid phase carrier, and the quenching product capturing nucleic acid has a first detectable marker selected from the group consisting of a fluorophore, a chemiluminescent label, a quantum dot, and a combination thereof ;
(b) a first probe;
(c) a second probe;
(d) a ligase for connecting the first probe and the second probe to form the third probe;
(e) a primer pair for amplifying the third probe, wherein the primer pair includes a first primer and a second primer, wherein at least one of the first primer and the second primer is a quenching primer, one end or one side of the quenching primer is connected with a quencher,
in addition, the quenching amplification product generated by the quenching primer through amplification and at least one of the quenching product capturing nucleic acid of the surface quantitation sub-detection region can be combined to form a double-stranded structure, and in the double-stranded structure, the quencher of the quenching amplification product causes the signal of the first detectable marker (such as fluorophore) of the capturing nucleic acid to be quenched.

The first probe has the structure of Formula II:

X₂'-T₂' (II)

wherein, X₂' is a reverse complement of the 5' end universal amplification primer;
T₂' is a reverse complement of the specific sequence at the 5' end of a targeting gene.

In a preferred embodiment, the length (nt) of X₂' is 15-50, preferably 19-36, more preferably 20-35.

In another preferred embodiment, the length (nt) of T₂' is 15-60, 19-36, more preferably 20-35.

The second probe has the structure of Formula III:

T₁'-P₁'-X₁' (III)

wherein, T₁' is a reverse complement of the specific sequence at the 3' end of a targeting gene;
P₁' is a barcode index sequence of a marker gene amplification combination;
X₁' is a reverse complement of the 3' end universal amplification primer.

In another preferred embodiment, the length (nt) of T₁' is 15-60, preferably 19-36, more preferably 20-35.

In another preferred embodiment, the length (nt) of P₁' is 10-500, preferably 19-100, more preferably 20-30.

In another preferred embodiment, the length (nt) of X₁' is 15-50, preferably 19-36, more preferably 20-35.

In another preferred embodiment, the ligase is selected from the group consisting of T4 ligase, NAD ligase (such as ligase-65), high temperature stable ligase (such as Taq DNA Ligase, HiFi Taq DNA Ligase, 9°N^{™} DNA Ligase), and a combination thereof.

In another preferred embodiment, the third probe has the structure of Formula IV:

X₂'-T₂'-T₁'-P₁'-X₁' (IV)

wherein, X₂' is a reverse complement of the 5' end universal amplification primer;
T₂' is a specific sequence at the 5' end of a targeting gene;
T₁' is a specific sequence at the 3' end of a targeting gene;
P₁' is a barcode index sequence that marks the gene amplification combination;
X₁' is a reverse complement of the 3' end universal amplification primer.

In another preferred embodiment, the first detectable marker is a fluorophore.

In another preferred embodiment, the fluorophore is selected from the group consisting of chemiluminescent label (luminescent labels).

In another preferred embodiment, the fluorophore is selected from the group consisting of quantum dots.

In another preferred embodiment, the ratio (Q1/QO) of the number Q1 of the quenching amplification product within the effective hybridization distance to the surface probe to the number Q0 of the corresponding surface probe (Q1/Q0) is 0.5 to 100, preferably 1-10.

In another preferred embodiment, the detection system further comprises one or more components selected from the group consisting of:
(c) buffers or buffer components for PCR amplification;
(d) polymerase used for PCR amplification.

In another preferred embodiment, the polymerase is selected from the group consisting of Taq enzyme, Pfu enzyme, Pwo enzyme, vent enzyme, KOD enzyme, superfi enzyme, and a combination thereof.

In another preferred embodiment, the length (bp) of the amplification product amplified by the first primer and the second primer is 50-2000, preferably 75-500, more preferably 75-150.

In another preferred embodiment, the quenching product capturing nucleic acids of different surface quantitation sub-detection regions are the same or different nucleic acid molecules.

In another preferred embodiment, the first probe and/or the second probe includes DNA, RNA, and a combination thereof.

In another preferred embodiment, the length (nt) of the first probe is 35-120, preferably 40-80, more preferably 45-65.

In another preferred embodiment, the length (nt) of the second probe is 40-160, preferably 55-120, more preferably 65-90.

In another preferred embodiment, the length (nt) of the third probe is 75-280, preferably 95-200, more preferably 110-155.

In another preferred embodiment, the length of the quenching product capturing nucleic acid is ≤ 150 nt, preferably ≤ 100 nt, more preferably ≤50 nt.

In another preferred embodiment, the length (nt) of the quenching primer is 15-50, preferably 19-36, more preferably 20-35.

In another preferred embodiment, the quenching primer capturing nucleic acid has a capture region that is substantially complementary or completely complementary to the quenching primer.

In another preferred embodiment, the quenching product capturing nucleic acid has the following structure of Formula I:

B0-B1-B2-B3 (I)

wherein,
B0 is a linking group connecting the quenching product capturing nucleic acid to the solid phase carrier;
B1 is no or a flexible transition region selected from the group consisting of: a flexible transition nucleic acid fragment with a length of 1-100 nt, a flexible transition polymer fragment with a length of 1-10 nm, and a combination thereof;
B2 is a capture region, wherein the sequence of the capture region is substantially complementary or completely complementary to the sequence of the quenching primer;
B3 is no or a 3' end sequence region of 1-20 nt in length.

In another preferred embodiment, the first detectable signal is located at B2 or B3, preferably at B2.

In another preferred embodiment, B3 is none.

In another preferred embodiment, one end or one side of the quenching primer capturing nucleic acid is connected with a first detectable marker (e.g., a fluorophore).

In another preferred embodiment, the other primer in the first primer and the second primer is a conventional primer or a universal primer.

In another preferred embodiment, on the solid phase carrier, the number of the surface quantitative sub-detection region is m, m is a positive integer of ≥ 1, and m≤n.

In another preferred embodiment, n is 2-500, preferably 5-250, more preferably 9-100 or 16-96.

In another preferred embodiment, m is 2-500, preferably 5-250, more preferably 9-100 or 16-96.

In another preferred embodiment, m=n.

In another preferred embodiment, the same first detectable marker is used in at least ≥2 (more preferably ≥3) surface quantitation sub-detection regions.

In another preferred embodiment, each of the surface quantitation sub-detection regions is used to detect different or the same target sequence.

In another preferred embodiment, each of the surface quantitation sub-detection regions is used to detect different target sequences.

In a second aspect of the present invention, it provides a method for quantitative PCR detection, comprising the steps of:
(a) providing a sample to be detected and the surface probe-based quantitative PCR detection system according to the first aspect of the present invention;
(b) in the presence of ligase and polymerase, under conditions suitable for ligation reaction and PCR amplification, using the quantitative PCR detection system to perform PCR amplification on the sample to be detected;
(c) detecting the signal of the first detectable marker of the one or more surface quantitation sub-detection regions on the solid support during or after the PCR amplification; and
(d) analyzing the detected signal of the first detectable marker to obtain a quantitative detection result of the sample to be detected.

In another preferred embodiment, in step (d), the analysis includes comparing the detected signal of the first detectable marker with a standard curve.

In another preferred embodiment, in step (d), the analysis includes comparing the detected signal of the first detectable marker with the detected signal before amplification.

In another preferred embodiment, the quantitative PCR detection is multiplex PCR detection.

In another preferred embodiment, the first detectable marker of each surface quantitation sub-detection region is the same.

In another preferred embodiment, the first detectable marker of each surface quantitation sub-detection region is different.

In a third aspect of the present invention, it provides a kit for quantitative PCR detection according to claim 10, comprising:
(a) a first container and a solid phase carrier located in the first container,
   one primary surface of the solid phase carrier is provided with n sub-detection regions, wherein n is a positive integer ≥ 2, and at least one sub-detection region is a surface quantitative sub-detection region;
   wherein, each of the surface quantitation sub-detection region is independently immobilized with a quenching product capturing nucleic acid, the quenching product capturing nucleic acid is a single-stranded nucleic acid, and one end of the quenching product capturing nucleic acid is immobilized on the surface of the solid phase carrier, and the quenching product capturing nucleic acid has a first detectable marker selected from the group consisting of a fluorophore, a chemiluminescent label (luminescent label), a quantum dot;
(b) a second container and a first probe, a second probe, and a ligase in the second container, the ligase ligating the first probe and the second probe into a third probe;
(c) a third container and a primer pair located in the third container, the primer pair being a primer pair for amplifying the third probe, wherein the primer pair includes a first primer and a second primer, wherein at least one of the first primer and the second primer is a quenching primer, and one end or one side of the quenching primer is connected with a quencher,
   in addition, the quenching amplification product generated by the quenching primer by amplifying the third probe and at least one of the quenching product capturing nucleic acid of the surface quantitation sub-detection region can be combined to form a double-stranded structure, and in the double-stranded structure, the quencher of the quenching amplification product causes the signal of the first detectable marker (such as fluorophore) of the capturing nucleic acid to be quenched;
(d) an optional fourth container and a buffer or buffer component for PCR amplification in the fourth container;
(e) an optional fifth container and a polymerase for PCR amplification in the fifth container; and
(f) optional instructions describing methods for quantitative PCR detection.

In another preferred embodiments, the first container, the second container, the third container, the fourth container, and the fifth container are the same container or different containers.

It should be understood that, within the scope of the present invention, each technical feature of the present invention described above and in the following (as examples) may be combined with each other to form a preferred technical solution, which is not listed here due to space limitations.

### Description of Figure

Figure 1 shows the reaction principle of traditional fluorescent quantitative PCR (qPCR).
Figure 2 shows the reaction principle of the present invention.
Figure 3 shows the detection chip and sub-detection region. The left is a schematic diagram of the detection chip, the upper left is a top view of the detection chip, and the lower left is a cross-sectional view of the detection chip structure. On the right is a photo of the 9 sub-detection regions on the chip.
Figure 4 shows a schematic diagram of the light path and temperature control of the detection device.
Figure 5 shows the detection results of the embodiment.
Figure 6 shows the verification experimental data of the multiplex reaction of the present invention for simultaneous real-time quantitative detection of multiple target sequences.

### DETAILED DESCRIPTION

After extensive and in-depth research, the inventors have developed for the first time a multiplex ligation-dependent amplification using only one pair of primers and multiple pairs of probes. At the same time of amplification, in each amplification cycle, the corresponding probes immobilized on the surface can quantitatively read the amplification products to achieve quantitative detection of specific sequences. In addition, even if only one or two or a few detectable markers and corresponding quencher are used, the detection effect of the present invention will not be affected. Therefore, the manufacturing cost and the use cost are greatly reduced. The present invention has been completed on this basis.

### Terms

As used herein, the term "primer" refers to an artificially synthesized short nucleic acid fragment (especially a DNA fragment) in the polymerase chain reaction that determines the initiation and termination positions required for amplification. "Universal primer", "unamplified primer" and "primer" are used interchangeably herein.

As used herein, the terms "quenching primer" or "quenching primer of the present invention" are used interchangeably and refer to a primer with a quencher. It is understood that either primer (e.g., a first primer or a second primer) or both primers of a primer pair can be the quenching primer.

As used herein, the terms "quenching amplification product", "amplification product", "quenching product", "quenching product of the present invention", "quenching amplification product of the present invention" are used interchangeably and refer to an amplification product with a quencher.

As used herein, the terms "quenching product capturing nucleic acid", "quenching product capturing nucleic acid of the present invention", "surface probe", "surface probe of the present invention" are used interchangeably and refer to a single-stranded nucleic acid molecule with a first detectable marker (such as a fluorophore) for capturing quenching amplification products and immobilized on the surface of a solid phase carrier. It should be understood that the quenching product capturing nucleic acids of the present invention are different from probes in the free state (which carry both a fluorophore and a quencher) in the prior art (e.g., fluorescence quantitative PCR).

As used herein, the terms "sub-detection region" and "array point" are used interchangeably.

### Detection system

The present invention provides a quantitative PCR detection system based on surface probes according to claim 1, and the detection system includes:
(a) a solid phase carrier, one primary surface of the solid phase carrier is provided with n sub-detection regions, wherein n is a positive integer ≥ 2, and at least one sub-detection region is a surface quantitative sub-detection region;
   wherein, each of the surface quantitation sub-detection region is independently immobilized with a quenching product capturing nucleic acid, the quenching product capturing nucleic acid is a single-stranded nucleic acid, and one end of the quenching product capturing nucleic acid is immobilized on the surface of the solid phase carrier, and the quenching product capturing nucleic acid has a first detectable marker selected from the group consisting of a fluorophore, a chemiluminescent label, a quantum dot, and a combination thereof ;
(b) a first probe;
(c) a second probe;
(d) a ligase for connecting the first probe and the second probe to form the third probe;
(e) a primer pair for amplifying the third probe, wherein the primer pair includes a first primer and a second primer, wherein at least one of the first primer and the second primer is a quenching primer, one end or one side of the quenching primer is connected with a quencher,
in addition, the quenching amplification product generated by the quenching primer through amplification and at least one of the quenching product capturing nucleic acid of the surface quantitation sub-detection region can be combined to form a double-stranded structure, and in the double-stranded structure, the quencher of the quenching amplification product causes the signal of the first detectable marker (such as fluorophore) of the capturing nucleic acid to be quenched.

In another preferred embodiment, the second primer may also have a quenching agent like the first primer, and the quenching amplification product produced by the second primer has corresponding different primer-capturing sub-detection regions on the surface.

In another preferred embodiment, both the first primer and the second primer have a quenching agent, but they correspond to one or more sub-detection regions at the same time, that is, each sub-detection region corresponding to the quenching product generated by the amplification of the pair of primers is simultaneously immobilized with the corresponding two amplification product capturing nucleic acids.

### Quenching product capturing nucleic acid, amplification primers and first and second probes

The first and second probes respectively have specific sequences T₂' and T₁' corresponding to the target nucleic acid sequence to be detected, and their design follows the traditional design principles of ligation probes, and its length is preferably 15-35 nucleotide units. A long specific sequence is beneficial to increase the specificity of recognition of the target to be detected. In the three-segment structure of the second probe, there is also a barcode index sequence P1' corresponding to the amplification product capturing nucleic acid, which is completely or partially identical to the quenching product capturing nucleic acid (i.e., surface probe) of a certain sub-detection region. The corresponding second probe for detecting each nucleic acid to be tested has its specific barcode index sequence Pi', which respectively corresponds to the surface probe on a certain sub-detection region.

It should be understood that although only the second probe has a complementary sequence corresponding to a surface probe in this description, the first probe may also have a complementary sequence corresponding to a surface probe, as long as the primer on its side is also a quenching primer.

In the present invention, for each target nucleic acid sequence to be detected, the same, usually the same two universal primers are used for specific amplification.

The two primers (primer pair) increase the specificity of the amplification reaction and accelerate the formation of amplification products, and the surface probe on the solid surface corresponding to Pi' with the barcode index sequence generated by it ensures the specificity and signal of detection. If both primers in a primer pair are quenching primers, the corresponding two surface probes can be respectively immobilized in different surface quantitation sub-detection regions, or they can be mixed and fixed in the same surface quantification sub-detection region.

The design of universal primers follows the traditional PCR primer design, and the length of the primer itself is preferably 20 - 35 nucleotide units. Short primers are beneficial to shorten the distance of the quenching amplification product from the fluorescent labels of the surface probe after hybridization with the surface probe.

It should be understood that although the label quenching agent is preferably at the 5' end of the quenching primer, the label can be conjugated or synthesized at any position on the quenching primer as long as it does not interfere with the amplification of the chain reaction and after hybridization with the surface probe, it is close enough to the fluorescent labels of the surface probe (the distance of less than 25 nucleotide units).

It should be understood that although the sequence P1' of the second probe is preferably completely complementary to the quenching product capturing nucleic acid on the surface, it can also be partially complementary, that is, it is complementary to a portion of the quenching amplification product generated by the quenching primer and the quenching product capturing nucleic acid (i.e., the surface probe).

However, the sequences of other amplification extensions with the amplification product should not be complementary to the surface probe and its other extensions should not exceed 150nt, and preferably should not exceed 120nt. The longer the amplification product is, the lower the hybridization efficiency of the surface probe is, so that the quenching of the fluorescence of the surface probe due to the hybridization of the quenching amplification product with the surface probe is not observed.

### solid phase carrier

In the present invention, the surface probes are immobilized on the surface quantitation sub-detection region of the solid phase carrier.

Typically, the 5' end of the surface probe is closer to the solid surface and the 3' end is further away from the solid surface.

### Surface probe quantitative PCR method

The present invention provides a surface probe quantitative PCR method.

In the present invention, if the target to be detected corresponding to the first probe and the second probe exists and the probe-substrate effective hybridization is formed, the first probe and the second probe will be ligated into the third probe by the ligase. Amplify the third probe with a universal amplification primer and generate a quenching amplification product containing the barcode index sequence. The core of the present invention is that the quenching amplification product hybridizes with the surface probe to gradually weaken the fluorescent signal of the surface probe to achieve real-time quantification of the target to be detected. In the method, the third probe is generated only when the target nucleic acid is present first. Before the third probe is amplified, the surface probe of the sub-detection region corresponding to barcode index P1' does not have any sequence complementary to it and has a quencher, and the surface probe of the sub-detection region is in the light-emitting state; after the third probe is amplified, the amplification product contains both the quenching agent of the quenching primer and the barcode index P1' because of the amplification and extension reaction. When the quenching amplification product is hybridized with the surface probe, as a result, the fluorescence of the surface probe is weakened, and the degree of fluorescence reduction is directly related to the concentration of the quenching amplification product, that is, the speed of the amplification reaction, so the target nucleic acid can be quantified.

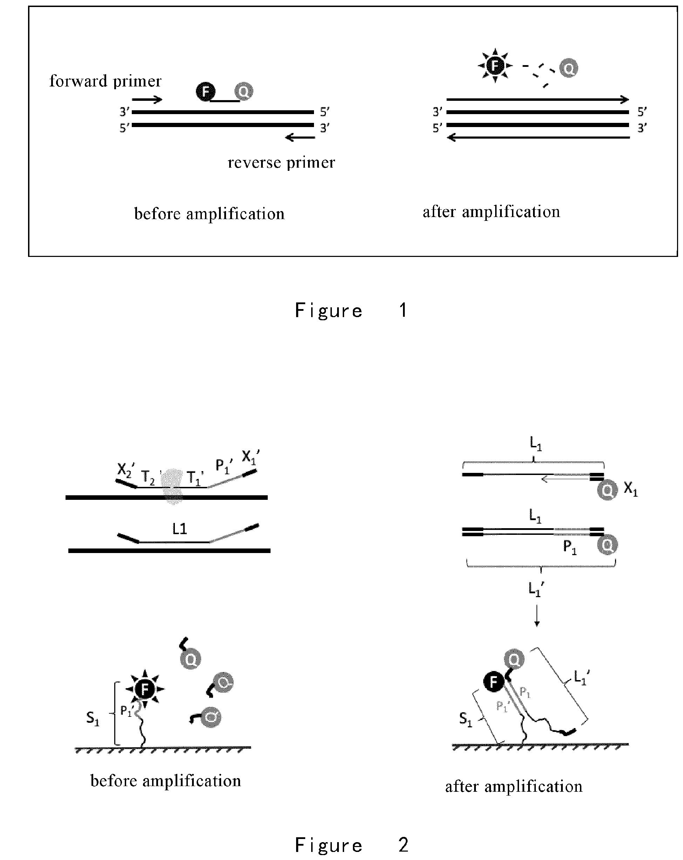

For ease of understanding, the present inventors describe in conjunction with the schematic diagram ( FIG. 2 ) and the prior art ( FIG. 1 ). It should be understood that the protection scope of the present invention is not limited by the described principles or schematic diagrams.

As shown in Figure 2, in the present invention, instead of using a solution probe with both a fluorescent agent and a quenching agent, quantitative PCR is achieved by using primers with only quenching agents and surface probes with fluorescent agents immobilized on the physical surface through the fluorescent probes on the physical surface.

Before amplification, there is no amplification product with a quenching agent in the solution to hybridize with the surface probe, so the probe points on the surface are in a light-emitting state.

When the target to be detected does exist in the reaction, a pair of "specific ligation probes" corresponding to the target, namely the first probe and the second probe (X₂'-T₂' and T₁'-P₁'-X₁') hybridize to the target, and the ligase links the two probes to form a sequence L₁ that can be amplified, that is, a third probe; if the detection target does not exist, its corresponding specific ligation probe, that is, the first probe and the second probe (X₂'-T₂' and T₁'-P₁'-X₁') will not be connected, and thus will not be amplified.

There is a quenching agent Q on the universal primer X₁, and by amplifying the ligated third probe, the amplification product therefore also carries the quenching agent Q. Specific ligation probes T₁'-P₁'-X₁' for each target to be detected, T₁' is the complementary sequence to the specific sequence of the target, P₁' (the sequence between T₁' and X₁' in the upper left of Figure 2 is not complementary to the target to be detected) is the marker (Barcode Index P₁') corresponding to a sub-detection region. After amplification, the amplification product contains Barcode Index P₁ and a quenching agent, which can hybridize with the corresponding surface probe S₁ on the surface (S1 contains a sequence that is completely complementary to P₁). The quenching agent Q carried by the amplimer has a FRET reaction with the fluorophore F of S₁, thus, the fluorescent signal of the surface probe is reduced. The weakening of the fluorescent signal directly corresponds to whether there is a target to be detected in the solution, and the intensity and speed of the signal weakening correspond to the initial concentration of the target to be detected.

In terms of multiplex detection, since the detection signal is derived from the surface probe, and because the physical position of the surface probe itself can be used to distinguish the corresponding target nucleic acid on it, multiplex detection can be achieved with only one label, without the multiple fluorophores of traditional multiplex qPCR. This method can be used for the quantitative detection of at least 1-100 target nucleic acids, or more than 100. The traditional multiplex qPCR technology is limited by the type of fluorescent agent and optical design, usually only 4-6 real-time quantitative detection can be performed, and very few can achieve 8.

In the present invention, it is very suitable for multiple quantitative detection, especially multiple real-time quantitative detection. In the present invention, the number of multiples is not particularly limited, and can be any positive integer ≥ 2, preferably 2-10000 or 2-500, preferably 3-250, more preferably 4-100 or 6-96 .

Typically, in the present invention, the signal reading of the surface probe can be performed for every cycle (or every 1 or every 2 cycles) at a selected time and temperature during the amplification reaction.

Before the third probe is amplified, the surface probe of the sub-detection region corresponding to Barcode Index P₁' does not have any sequence complementary to it and has a quencher, and the surface probe of the sub-detection region is in a light-emitting state; After the third probe is amplified, the amplification product not only contains the quenching agent of the quenching primer, but also contains the Barcode Index P₁ because of the amplification and extension reaction. When the quenching amplification product is hybridized with the surface probe, as a result, the fluorescence of the surface probe is weakened, and the degree of fluorescence weakening is directly related to the concentration of the quenching amplification product, that is, the speed of the amplification reaction, so the target nucleic acid can be quantified.

### Kit

The present invention also provides a kit for quantitative PCR detection according to claim 10, which includes:
(a) a first container and a solid phase carrier located in the first container,
   one primary surface of the solid phase carrier is provided with n sub-detection regions, wherein n is a positive integer ≥ 2, and at least one sub-detection region is a surface quantitative sub-detection region;
   wherein, each of the surface quantitation sub-detection region is independently immobilized with a quenching product capturing nucleic acid, the quenching product capturing nucleic acid is a single-stranded nucleic acid, and one end of the quenching product capturing nucleic acid is immobilized on the surface of the solid phase carrier, and the quenching product capturing nucleic acid has a first detectable marker selected from the group consisting of a fluorophore, a chemiluminescent label (luminescent label), a quantum dot;
(b) a first probe;
(c) a second probe;
(d) a ligase ligating the first probe and the second probe into a third probe;
(e) a primer pair that matches the quenching product capturing nucleic acid and is used to amplify the third probe, wherein the primer pair includes a first primer and a second primer, wherein at least one primer in the first primer and the second primer is a quenching primer, and one end or one side of the quenching primer is connected with a quencher,
   in addition, the quenching amplification product generated by the amplification of the quenching primer and the third probe and the quenching product capturing nucleic acid of the at least one surface quantitation detection region can be combined to form a double-stranded structure, and in the double-stranded structure , the quencher of the quenching product causes the signal of the first detectable marker (such as a fluorophore) of the capturing nucleic acid to be quenched;
(d) an optional fourth container and a buffer or buffer component for PCR amplification in the fourth container;
(e) an optional fifth container and a polymerase for PCR amplification in the fifth container; and
(f) optional instructions describing methods for quantitative PCR detection.

In addition, the kit of the present invention may also contain other compositions or components, such as standard curves, quality control samples, and the like.

In the present invention, any two, three or all of the first, second, third, fourth, and fifth containers are the same container.

In another preferred embodiment, the first, second, third, fourth and fifth containers are different containers.

In one embodiment, all of the reagents themselves may be present in the first container.

In another preferred embodiment, all reagents (enzymes, primers, salts) are stored in the first container in a lyophilized manner, the DNA to be detected is thawed in the corresponding buffer, and injected into the first container during the reaction.

### Reaction devices and detection system

The present invention also provides a reaction device and a detection system for quantitative PCR based on surface probes.

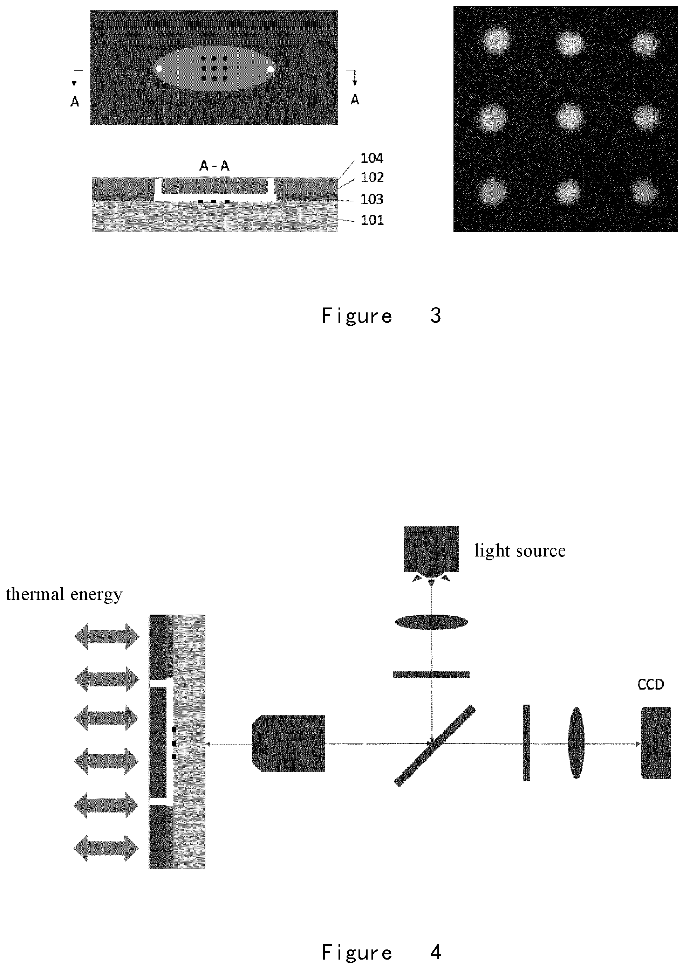

A representative reaction device is shown in Figure 3.

In the present invention, the chip used for the reaction can be in any form, as long as the reaction chamber has an inner surface, a microarray (surface quantitation sub-detection region array) can be prepared in advance and subsequent fluorescence signal reading can be performed.

As shown in Figure 3, a typical design is a flat reaction chamber design.

The reaction chamber is shown in Figure 3 and consists of three parts. The first part 101 is a piece of plastic or glass, the second part 102 is another piece of flat plastic or glass, and combined with the third part 103, a double-sided tape with a thickness of 0.25 mm. The double-sided tape has a cut portion to form a reaction chamber. The reaction chamber can also be composed of only two parts: the first part already contains the grooves that constitute the reaction chamber, and the second part can be provided with single-sided adhesive, which can directly bond the first part, or it can also form a closed reaction chamber with the first part by means of ultrasonic fusion, thermal fusion, double-sided adhesive or ultraviolet curing adhesive. The chamber has inlet and outlet channels or inlets and outlets for the reaction solution to flow in. After the reaction solution is added, the valve on the channel can be closed or the inlet and outlet can be permanently closed. In this example, a single-sided adhesive 104 with a thickness of 0.1 mm is used to permanently close the entrance and exit.

An inner surface of the reaction chamber was chemically treated and spotted with a microarray (surface quantitation sub-detection region array) before the reaction chip was integrated. As an example, Figure 3 shows a 3x3 microarray. The array is used to detect 3 different nucleic acids (2 target nucleic acids and 1 internal reference), and corresponding to each nucleic acid sequence to be detected, there are three sub-detection regions immobilized with surface probes corresponding to the nucleic acid to be detected. Detection using more than one sub-detection region for each nucleic acid sequence to be detected, when the detection signal of one of the sub-detection regions is disturbed by air bubbles or impurities, there are still other spots with the same sequence to confirm the target nucleic acid to be detected. The number of sub-detection regions constituting the microarray can be 2-100, or more than 100.

The material on one side of the reaction chamber must have sufficient transmittance in the relevant wavelength band (excitation and emission) of the fluorescent agent, with a transmittance of at least 80%, preferably more than 90%; at the same time, the lower the autofluorescence of the material on this side, the better. In this example, the first part of the reaction chamber is a glass slide with low autofluorescence (BOROFLOAT^{®}33 from Schott Company), which has a transmittance of more than 90% to visible light, and its autofluorescence is much lower than that of ordinary glass materials.

Surface nucleic acid probe sequences are typically coupled to the solid surface via a short linker.

In the present invention, the linker can be oligonucleotide (oligo), polymer (polymer) (e.g. PEG), or a combination.

The core of the present invention is that the hybridization of the quenching amplification product and the surface probe causes the fluorescence of the surface probe to be quenched due to the FRET reaction. In order to improve the hybridization efficiency, the length of the conjugation linkers (linker) should not be less than 5 nm, preferably more than 10 nm, so that the surface probe available for hybridization is far enough away from the solid surface that the quenching amplification product can fully hybridize therewith. Solid surfaces are often surface chemically treated to generate reactive groups such as NHS esters, thioesters, etc., which can react with conjugation linkers. The link between the conjugation linkers and the solid surface is thermally stable and does not break or fall off due to the cooling and heating cycles of PCR. Slides with NHS esters available for conjugation on the surface (available from ArrayIt and others) are used in this example. The surface probe is a synthetic sequence, and an amino group is synthesized at the 5' end that can be covalently coupled to the NHS ester on the surface of the glass slide. There is a conjugation linker Polyethylene glycol chain [PEG]₅₀ (polyethylene glycol) between the amino group and the DNA sequence. The sequence of the deoxyribonucleic acid is the same as the barcode index Pᵢ' on the second probe of each target to be tested (that is, complementary to its corresponding amplification product), and is coupled to the fluorescent agent Cy3 at its 3' end.

Surface probe arrays (i.e., sub-detection region arrays) can employ conventional methods for generating microarrays. Scienion's non-contact spotting machine is used for spotting in this example. Touch-based spotting machines such as ArrayIt's SpotBot^{®} are also a common tool. In this example, the diameter of the array points (i.e. each sub-detection region) is about 50 µm, and the edge-to-edge spacing of each point is about 100 µm.

The total number of each surface probe should be equivalent to the number of the corresponding final amplification products in the solution near the array point, so that when the quenching amplification products in the solution near the array point increase due to amplification, on the array points, surface probes will gradually hybridize with the quenching amplification products, resulting in a gradual decrease in the fluorescent signal until almost complete annihilation at the end. The surface probe density per spot on the microarray should be higher than 500 fmole/cm², preferably higher than 2000 fmole/cm². Before amplification, there is no DNA sequence with a quenching agent to hybridize with the surface probe, the fluorescent agent on the surface probe is in a fully light-emitting state, and the fluorescence brightness of the array point is the highest. When the corresponding target nucleic acid exists and the third probe generated by ligase ligation is amplified, the quenching primer is extended (amplified), and the extended part contains the Barcode Index Pᵢ sequence, and its corresponding surface probe will hybridize to it, resulting in the quenching of the fluorescence of the surface probe, when the quenching amplification product reaches the maximum value, the fluorescence brightness of the array point reaches the minimum value. During the cycle, with the increase of quenching amplification products, the self-fluorescence signal of the surface probe gradually decreases due to its hybridization. If the concentration of the initial target nucleic acid is relatively low, the growth of the quenching amplification product will be relatively late and slow, so the fluorescence decrease of the surface probe will also occur relatively late and slow. There is a one-to-one correspondence and monotonic decreasing relationship between the fluorescence brightness of the array point and the number of quenching amplification products, so the degree and speed of the fluorescence change of each array point can be used to quantify its corresponding initial target nucleic acid concentration.

The heating and cooling cycle control of PCR can be performed on one or both sides of the reaction chamber simultaneously. Simultaneous temperature control on both sides can increase the rate of temperature regulation in the reaction chamber, thereby shortening the reaction time. For the sake of example, the preset here is for the heating-cooling cycle only from one side. Whether it is single-sided or double-sided for temperature control, the thinner the reaction chamber, the faster the temperature adjustment, and the easier it is for the reaction chamber solution to reach thermal equilibrium at each temperature node. Therefore, the thickness of the reaction chamber is usually below 2mm, preferably below 1mm. The example uses a 0.25mm design. The thickness of the solid material on the temperature control side ensures the strength of the chip, and the thinner the material, the faster the heating and cooling cycle. In this example a 0.5mm polycarbonate sheet. In addition, since the reading of the fluorescence signal is performed from the glass side, black polycarbonate is used here to further reduce the background noise during fluorescence reading.

There are many common methods of heating and cooling cycles in PCR, such as using a thermoelectric module, a water bath, an oil bath to control a metal module in contact with the reaction chamber, or directly heating the solution with infrared rays. In this example, a computer-controlled thermoelectric module heats and cools a copper block, which has high thermal conductivity so that the reaction chamber can reach thermal equilibrium faster with each temperature change.

The microarray fluorescence signal reading on the inner surface of the reaction chamber can use a common monochromatic fluorescence signal reading system. Figure 4 depicts a typical monochromatic fluorescence signal reading system. In this example, a fluorescence microscope (Olympus IX73) was used to collect the fluorescence signals of the surface probes on the microarray, the principle of which is consistent with the system described in the above figure.

### application

The method of the present invention is particularly advantageous for multiplex quantitative detection of DNA.

If traditional qPCR needs to achieve multiple detection, each marker to be tested needs to be equipped with a different fluorescent agent, which makes the optical design of the device very complicated, and the spectrum is limited, and the multiplicity that can be achieved is limited. The present invention transfers the signal to be detected from the solution to the physical surface, and multiple positions can be spotted on the physical surface, and each spot can can design different surface probe sequences and solution probes (i.e., the first and second probes) for different targets to be tested, but each spot can use the same fluorophore, enabling multiple quantitative detection under one fluorophore. Ideally, it supports 2X-200X, and can also support multiple detections of 200-1,000X or 1,000-10,000X.

In terms of application fields or occasions, the present invention can be used for the identification of microorganisms. For example, in the case of upper respiratory tract infections, quantitative detection can be performed by designing multiple surface probes and solution probes (i.e., first and second probes) against specific sequences of common viruses and bacteria to identify specific source of microbial infection. The invention can also identify the subtypes of microorganisms, for example, multiple surface probes can be designed for quantitative detection of specific sequences of different subtypes of HPV, and HPV samples can be typed in one reaction.

The main advantages of the present invention include:
(a) Only one pair of primers and multiple pairs of probes are used to perform multiple ligation-dependent amplification. At the same time of amplification, the corresponding probes immobilized on the surface can quantitatively read the amplification products during each amplification cycle to achieve quantitative detection of specific sequences.
(b) Quantitative analysis can be performed quickly and easily.
(c) A large number of target sequences can be detected simultaneously in parallel.
(d) Only one or two detectable markers and corresponding quencher need to be used, so that both the manufacturing cost and the use cost are greatly reduced.
(e) The detection system has strong anti-interference ability.
(f) Even if only one or two or a few detectable markers and corresponding quencher are used, the detection effect of the present invention will not be affected, thus greatly reducing both the manufacturing cost and the use cost.
(g) The detection background of the present invention is low, that is, the detection signal-to-noise ratio is good. In the present invention, because the primer label in the solution is a quenching agent, and the solution itself does not have any fluorescent agent (even temporarily suppressed fluorescent agents). Therefore, the background light noise around the surface quantitation sub-detection region during detection is not sensitive to the concentration of primers and the amount of primers. There are temporarily inhibited fluorescent agents in the reaction solution of traditional qPCR, because the fluorescence inhibition effect cannot be 100%, especially when the TaqMan probe does not have a hairpin structure, the inhibition efficiency is lower, so that the background light noise of traditional qPCR is directly related to the probe concentration.
(h) The primer of the present invention is a universal primer, and the surface probe is used as a barcode index and has nothing to do with the target sequence to be detected. When different target panels need to be detected, there is no need to replace the primers and surface probes (so there is no need to replace the chip), only the first and second probes (that is, to replace the specific sequence Tᵢ') need to be replaced. This feature enables multiple detection kits to share chips and most of the reagents, making full use of the advantages of large-scale manufacturing, thereby greatly reducing the cost of the kits.

The present invention will be further described below in conjunction with specific embodiments. It should be understood that these examples are only used to illustrate the present invention and not to limit the scope of the present invention. The experimental method of unreceipted specific conditions in the following examples, usually according to conventional conditions, such as people such as Sambrook, molecular cloning: conditions described in laboratory manual (New York: Cold Spring Harbor Laboratory Press, 1989), or according to manufacture conditions recommended by the manufacturer. Percentages and parts are weight percentages and parts unless otherwise specified.

Unless otherwise specified, the materials and reagents used in the examples of the present invention are all commercially available products.

### Example 1 Quantitative PCR reaction and detection based on surface probes

### 2.1 Reactants in the amplification reaction:

RNA extracted from bordetella pertussis (purchased from ATCC, product number 9797DQ)

### 2.2 Methods

A two-step linking reaction is used (three steps, i.e., annealing and linking in two steps are equally applicable).

The Ligation reaction is set to:
1. Thermal denaturation, 95 °C, 30 seconds
2. annealing and linking, 60 °C, 15 minutes.

Two-step PCR is then used (Traditional three-step PCR also works). In the three-step PCR, the reading of the fluorescent signal can be selected in the annealing or amplification (extension) period, preferably in the amplification period. The PCR temperature is set to:
1. Heat denaturation, 95 °C, 5 minutes
2. 40 thermal cycles, each cycle includes:
   a. 95°C, 5 seconds
   b. 60°C, 60 seconds (wherein, the fluorescence signal of the microarray is read in the last 10 seconds of 60°C)

### 2.3 Primers and probes

Universal primers used to amplify the third probe corresponding to the first target to be tested (the specific sequence of the toxin promoter region of bordetella pertussis):
X2' (M13-47 Forward): GTC GTG ACT GGG AAA ACC CTG GCG (SEQ ID NO.: 1)
X1' (M13-48 Reverse): BHQ/AGC GGA TAA CAA TTT CAC ACA GG (SEQ ID NO.: 2).
Amplification product length = 118 bp
X1 is a quenching primer, and the quencher is BHQ (Black hole quencher).

T₁'/T₂': the reverse complement containing the specific sequence of the toxin promoter region of bordetella pertussis
T₁': GAC AGG CTG TAC CTA CGG TTT AA (SEQ ID NO.: 3) (23bp)
T₂': TTG AAA CAA GCC GAC GCA ACG (SEQ ID NO.: 4) (21 bp)

P₁': Barcode Index 1
P₁': GTA ACT CTA CAG ACG GAC GTA GTC A (SEQ ID NO.: 5) (25bp)

The first probe (X2'-T2') is GTC GTG ACT GGG AAA ACC CTG GCG TTG AAA CAA GCC GAC GCA ACG (SEQ ID NO.: 6).

The second probe (T1'-P1'-X1') is GAC AGG CTG TAC CTA CGG TTT AAG TAA CTC TAC AGA CGG ACG TAG TCA CCT GTG TGA AAT TGT TAT CCG CT (SEQ ID NO.:7).

The surface probe is (NH₂)₁₂-(PEG)₅₀-GTA ACT CTA CAG ACG GAC GTA GTC A (SEQ ID NO.: 8) - Cy3.

The corresponding primers used to amplify the second target to be tested (human BFL1 specific sequence):
X1, X2 are universal primers, the same as the first target. Similarly, X1 is a quenching primer, and the quencher is BHQ (Black hole quencher).

T₁'/T₂': contains the reverse complement of the BFL1-specific sequence
T₁': AGA TAG TCC TGA GCC AGC CTG T (SEQ ID NO.: 9) (22bp)
T₂': GTG GTA TCT GTA GGA CGC ACT GC (SEQ ID NO.: 10) (23bp)

P₁': Barcode Index 2
P₁': CCC TCA ACG GTA TCG CGT CGG TTG C (SEQ ID NO.: 11) (25bp)

The first probe (X2'-T2') is GTC GTG ACT GGG AAA ACC CTG GCG GTG GTA TCT GTA GGA CGC ACT GC (SEQ ID NO.: 12).

The second probe (T1'-P1'-X1') is AGA TAG TCC TGA GCC AGC CTG TC CCT CAA CGG TAT CGC GTC GGT TGC CCT GTG TGA AAT TGT TAT CCG CT (SEQ ID NO.: 13).

The surface probe is (NH₂)₁₂-(PEG)₅₀- CCC TCA ACG GTA TCG CGT CGG TTG C (SEQ ID NO.: 14)- Cy3.

Ligation reaction: denaturation at 95°C for 30 seconds, reaction at 60°C for 15 minutes to complete annealing and ligation in one step. The reaction system includes HiFi Taq DNA Ligase Buffer (NEB #M0647), HiFi Taq DNA Ligase (NEB #M0647s) ligase, the first probe X₂'-T₂' (2nM), the second probe X₁'-P₁'- T₁' (2nM). The target nucleic acid of the sample to be tested is added to the positive reaction; the target nucleic acid of the sample to be tested is not added to the negative reaction. The ligation reaction is terminated by heating at 95°C for 5 minutes, and the DNA polymerase is activated at the same time.

PCR amplification reaction, the amplification reagent mixture comprises:
- Standard PCR reagents: 1X Fast Start Taq (0.2 unit/ul), 2 mM MgCl₂, 0.5 mg/ml BSA, 1x Fast Start buffer
- Amplification Primer X1 (250nM) with BHQ (Black Hole Quencher)
- Amplification Primer X2 (250nM)

40 amplification cycles are performed: 95 °C for 15 seconds, 60 °C for 60 seconds.

### 2.4 Solid phase carrier

The reaction chip as shown in Figure 3, including
- Slides with NHS esters available for conjugation
- There are microscale surface probe lattices on the glass slide, and the surface probes are immobilized on the glass slide by covalent coupling with the surface NHS esters through the amino group at the 5' end.
- Double-sided tape with a thickness of 0.25mm cut in the middle
- 0.5mm thick black polycarbonate sheet
- Single-sided tape with a thickness of 0.1mm

### 2.5 Results

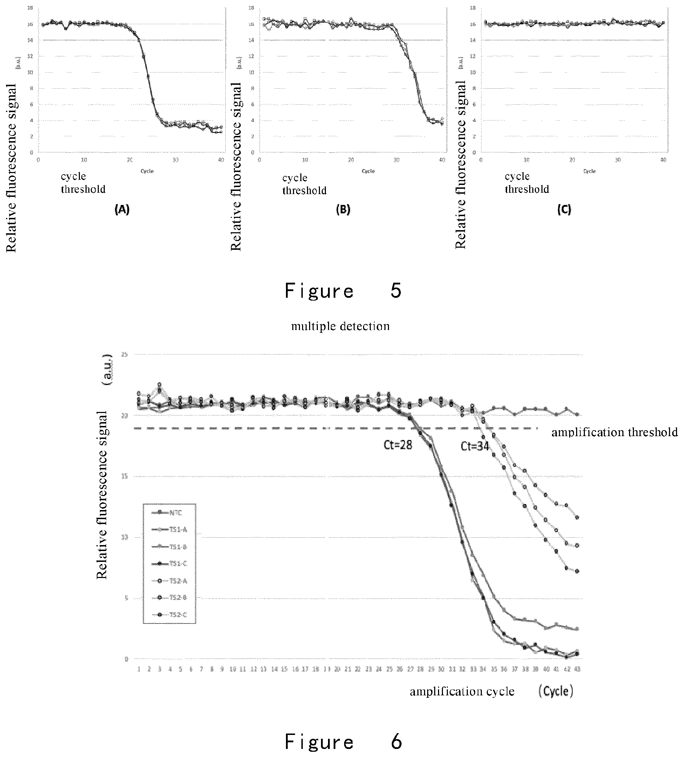

DNA ligation and polymerization are carried out under the above reaction conditions, and the results of quantitative positive and negative reactions are shown in Figure 5. Each curve as shown in the figure is the fluorescence brightness curve of each detection point (sub-detection region) in each PCR cycle. Figure 5a shows the result of adding the target nucleic acid (10⁵ copies) of the sample to be detected in the reaction. It can be seen that the fluorescence brightness of each detection point (3 in total) corresponding to the target to be detected gradually decreases with the amplification reaction progresses, indicating that the target to be tested is detected; the fluorescence intensity of the detection point crosses a set threshold near the 23rd cycle. Figure 5b shows the result of adding the target nucleic acid (500 copies) of the sample to be detected in the reaction. It can be seen that the fluorescence brightness of each detection point corresponding to the target to be detected (3 in total) gradually decreases with the progress of the amplification reaction, indicating that the target to be tested is detected; the fluorescence intensity of the detection point crosses the same set threshold around the 30th to 31st cycle. Figure 5c shows that the sample target to be detected is not added to the reaction. It can be seen that the fluorescence brightness of each detection point corresponding to the target to be detected (3 in total) has no obvious change with the progress of the amplification reaction, and has never exceeded the set threshold, indicating that the target to be tested is not detected.

FIG. 6 is the verification experimental data of the multiplex reaction of the present invention for simultaneous real-time quantitative detection of multiple target sequences. Each curve shown in the figure is also the fluorescence brightness curve of each detection point (sub-detection region) in each PCR cycle. In the figure, NTC (No Template Control) is a negative control, the same reaction as above, there is no significant amplification reaction at the NTC detection point, and the fluorescent signal never exceeds the threshold. The first target (TargetSubstrate1) is the pertussis target (2000copies) of the same experiment in Figure 5a. The fluorescence brightness of the detection point corresponding to the target TS1-A/B/C gradually decreased with the progress of the amplification reaction, indicating that the target to be tested is detected; the fluorescence intensity of the detection point has exceeded the set threshold around the 28th cycle. At the same time, the reaction also contains 300 copies of the second target (TargetSubstrate2). It can be seen that the fluorescence brightness of each detection point (TS2-A/B/C) corresponding to the target to be tested gradually decreases with the progress of the amplification reaction. The same set thresholds are crossed around the 34th to 35th cycles.

The results show that the method of the present invention can be used to detect the target nucleic acid to be detected, and the concentration of the target nucleic acid to be detected can be determined according to the fluorescence change speed of the detection point.

## Claims

1. A quantitative PCR detection system based on a surface probe, the detection system comprises:
(a) a solid phase carrier, one primary surface of the solid phase carrier is provided with n sub-detection regions, wherein n is a positive integer ≥ 2, and at least one sub-detection region is a surface quantitative sub-detection region;
wherein, each of the surface quantitation sub-detection region is independently immobilized with a quenching product capturing nucleic acid, the quenching product capturing nucleic acid is a single-stranded nucleic acid, and one end of the quenching product capturing nucleic acid is immobilized on the surface of the solid phase carrier, and the quenching product capturing nucleic acid has a first detectable marker selected from the group consisting of a fluorophore, a chemiluminescent label, a quantum dot, and a combination thereof ;
(b) a first probe;
(c) a second probe;
(d) a ligase for connecting the first probe and the second probe to form the third probe;
(e) a primer pair for amplifying the third probe, wherein the primer pair includes a first primer and a second primer, wherein at least one of the first primer and the second primer is a quenching primer, one end or one side of the quenching primer is connected with a quencher,
in addition, the quenching amplification product generated by the quenching primer through amplification and at least one of the quenching product capturing nucleic acid of the surface quantitation sub-detection region can be combined to form a double-stranded structure, and in the double-stranded structure, the quencher of the quenching amplification product causes the signal of the first detectable marker (such as fluorophore) of the capturing nucleic acid to be quenched;
wherein the first probe has the structure of Formula II:
X₂'-T₂' (II)
wherein, X₂' is a reverse complement of the 5' end universal amplification primer;
T₂' is a reverse complement of the specific sequence at the 5' end of a targeting gene;
wherein the second probe has the structure of Formula III:
T₁'-P₁'-X₁' (III)
wherein, T₁' is a reverse complement of the specific sequence at the 3' end of a targeting gene;
P₁' is a barcode index sequence of a marker gene amplification combination;
X₁' is a reverse complement of the 3' end universal amplification primer.

2. The detection system of claim 1, wherein the ratio (Q1/Q0) of the number Q1 of the quenching amplification product within the effective hybridization distance to the surface probe to the number Q0 of the corresponding surface probe (Q1/Q0) is 0.5 to 100, preferably 1-10.

3. The detection system of claim 1, wherein the length (nt) of X₂' is 15-50, preferably 19-36, more preferably 20-35.

4. The detection system of claim 1, wherein the length (nt) of T₂' is 15-60, 19-36, more preferably 20-35.

5. The detection system of claim 1, wherein the quenching product capturing nucleic acid has the following structure of Formula I:
B0-B1-B2-B3 (I)
wherein,
B0 is a linking group connecting the quenching product capturing nucleic acid to the solid phase carrier;
B1 is no or a flexible transition region selected from the group consisting of: a flexible transition nucleic acid fragment with a length of 1-100 nt, a flexible transition polymer fragment with a length of 1-10 nm, and a combination thereof;
B2 is a capture region, wherein the sequence of the capture region is substantially complementary or completely complementary to the sequence of the quenching primer;
B3 is no or a 3' end sequence region of 1-20 nt in length.

6. The detection system of claim 1, wherein the length (nt) of T₁' is 15-60, preferably 19-36, more preferably 20-35.

7. The detection system of claim 1, wherein the length (nt) of P₁' is 10-500, preferably 19-100, more preferably 20-30.

8. The detection system of claim 1, wherein the third probe has the structure of Formula IV:
X₂'-T₂'-T₁'-P₁'-X₁' (IV)
wherein, X₂' is a reverse complement of the 5' end universal amplification primer;
T₂' is a specific sequence at the 5' end of a targeting gene;
T₁' is a specific sequence at the 3' end of a targeting gene;
P₁' is a barcode index sequence that marks the gene amplification combination;
X₁' is a reverse complement of the 3' end universal amplification primer.

9. A method for quantitative PCR detection, comprising the steps of:
(a) providing a sample to be detected and the surface probe-based quantitative PCR detection system of claim 1;
(b) in the presence of ligase and polymerase, under conditions suitable for ligation reaction and PCR amplification, using the quantitative PCR detection system to perform PCR amplification on the sample to be detected;
(c) detecting the signal of the first detectable marker of the one or more surface quantitation sub-detection regions on the solid support during or after the PCR amplification; and
(d) analyzing the detected signal of the first detectable marker to obtain a quantitative detection result of the sample to be detected.

10. A kit for quantitative PCR detection, comprising:
(a) a first container and a solid phase carrier located in the first container,
one primary surface of the solid phase carrier is provided with n sub-detection regions, wherein n is a positive integer ≥ 2, and at least one sub-detection region is a surface quantitative sub-detection region;
wherein, each of the surface quantitation sub-detection region is independently immobilized with a quenching product capturing nucleic acid, the quenching product capturing nucleic acid is a single-stranded nucleic acid, and one end of the quenching product capturing nucleic acid is immobilized on the surface of the solid phase carrier, and the quenching product capturing nucleic acid has a first detectable marker selected from the group consisting of a fluorophore, a chemiluminescent label (luminescent label), a quantum dot;
(b) a second container and a first probe, a second probe, and a ligase in the second container, the ligase ligating the first probe and the second probe into a third probe;
(c) a third container and a primer pair located in the third container, the primer pair being a primer pair for amplifying the third probe, wherein the primer pair includes a first primer and a second primer, wherein at least one of the first primer and the second primer is a quenching primer, and one end or one side of the quenching primer is connected with a quencher,
in addition, the quenching amplification product generated by the quenching primer by amplifying the third probe and at least one of the quenching product capturing nucleic acid of the surface quantitation sub-detection region can be combined to form a double-stranded structure, and in the double-stranded structure, the quencher of the quenching amplification product causes the signal of the first detectable marker (such as fluorophore) of the capturing nucleic acid to be quenched;
(d) an optional fourth container and a buffer or buffer component for PCR amplification in the fourth container;
(e) an optional fifth container and a polymerase for PCR amplification in the fifth container; and
(f) optional instructions describing methods for quantitative PCR detection;
wherein the first probe has the structure of Formula II:
X₂'-T₂' (II)
wherein, X₂' is a reverse complement of the 5' end universal amplification primer;
T₂' is a reverse complement of the specific sequence at the 5' end of a targeting gene;
wherein the second probe has the structure of Formula III:
T₁'-P₁'-X₁' (III)
wherein, T₁' is a reverse complement of the specific sequence at the 3' end of a targeting gene;
P₁' is a barcode index sequence of a marker gene amplification combination;
X₁' is a reverse complement of the 3' end universal amplification primer.

## Patentansprüche

1. Quantitatives PCR-Nachweissystem auf der Basis einer Oberflächensonde, wobei das Nachweissystem Folgendes umfasst:
(a) einen Festphasenträger, wobei eine Primärfläche des Festphasenträgers mit n Teilnachweisbereichen versehen ist, wobei n eine positive ganze Zahl ≥ 2 ist und wenigstens ein Teilnachweisbereich ein flächenquantitativer Teilnachweisbereich ist;
wobei jeder der flächenquantitativen Teilnachweisbereiche unabhängig mit einer Quenchingprodukt-abfangenden Nucleinsäure immobilisiert ist, die Quenchingprodukt-abfangenden Nucleinsäure eine einzelsträngige Nucleinsäure ist und ein Ende der Quenchingprodukt-abfangenden Nucleinsäure auf der Oberfläche des Festphasenträgers immobilisiert ist und die Quenchingprodukt-abfangenden Nucleinsäure einen ersten nachweisbaren Marker aufweist, der aus der Gruppe ausgewählt ist, die aus einem Fluorophor, einem Chemilumineszenzmarker, einem Quantenpunkt und einer Kombination davon besteht;
(b) eine erste Sonde;
(c) eine zweite Sonde;
(d) eine Ligase zum Verbinden der ersten Sonde mit der zweiten Sonde unter Bildung der dritten Sonde;
(e) ein Primerpaar zum Amplifizieren der dritten Sonde, wobei das Primerpaar einen ersten Primer und einen zweiten Primer umfasst, wobei der erste Primer und/oder der zweite Primer ein quenchender Primer ist, ein Ende oder eine Seite des quenchenden Primers mit einem Quencher verbunden ist,
außerdem das durch den quenchenden Primer durch Amplifikation erzeugte quenchende Amplifikationsprodukt und wenigstens eine der Quenchingprodukt-abfangenden Nucleinsäuren des flächenquantitativen Teilnachweisbereichs unter Bildung einer doppelsträngigen Struktur miteinander kombiniert sein können und in der doppelsträngigen Struktur der Quencher des quenchenden Amplifikationsprodukts bewirkt, dass das Signal des ersten nachweisbaren Markers (wie eines Fluorophors) der abfangenden Nucleinsäure gelöscht wird;
wobei die erste Sonde die Struktur von Formel II aufweist:
X₂'-T₂' (II)
wobei X₂' ein reverses Komplement des 5'-Ende-Universalamplifikationsprimers ist;
T₂' ein reverses Komplement der spezifischen Sequenz am 5'-Ende eines Targeting-Gens ist;
wobei die zweite Sonde die Struktur von Formel III aufweist:
T₁'-P₁'-X₁' (III)
wobei T₁' ein reverses Komplement der spezifischen Sequenz am 3'-Ende eines Targeting-Gens ist;
P₁' eine Strichcode-Indexsequenz einer Marker-Gen-Amplifikationskombination ist;
X₁' ein reverses Komplement des 3'-Ende-Universalamplifikationsprimers ist.

2. Nachweissystem gemäß Anspruch 1, wobei das Verhältnis (Q1/Q0) der Anzahl Q1 des quenchenden Amplifikationsprodukts innerhalb des effektiven Hybridisierungsabstands von der Oberflächensonde zu der Anzahl Q0 der entsprechenden Oberflächensonde (Q1/Q0) 0,5 bis 100, vorzugsweise 1-10, beträgt.

3. Nachweissystem gemäß Anspruch 1, wobei die Länge (nt) von X₂' 15-50, vorzugsweise 19-36, besonders bevorzugt 20-35, beträgt.

4. Nachweissystem gemäß Anspruch 1, wobei die Länge (nt) von T₂' 15-60, 19-36, besonders bevorzugt 20-35, beträgt.

5. Nachweissystem gemäß Anspruch 1, wobei die Quenchingprodukt-abfangenden Nucleinsäure die folgende Struktur der Formel I aufweist:
B0-B1-B2-B3 (I)
wobei
B0 eine Verknüpfungsgruppe ist, die die Quenchingprodukt-abfangenden Nucleinsäure mit dem Festphasenträger verbindet;
B1 kein oder ein flexibler Übergangsbereich ist, der aus der Gruppe ausgewählt ist, die aus Folgenden besteht: einem flexiblen Übergangsnucleinsäurefragment mit einer Länge von 1-100 nt, einem flexiblen Übergangspolymerfragment mit einer Länge von 1-10 nm und einer Kombination davon;
B2 ein Abfangbereich ist, wobei die Sequenz des Abfangbereichs im Wesentlichen komplementär oder vollständig komplementär zu der Sequenz des quenchenden Primers ist;
B3 kein oder ein 3'-Ende-Sequenzbereich mit einer Länge von 1-20 nt ist.

6. Nachweissystem gemäß Anspruch 1, wobei die Länge (nt) von T₁' 15-60, vorzugsweise 19-36, besonders bevorzugt 20-35, beträgt.

7. Nachweissystem gemäß Anspruch 1, wobei die Länge (nt) von P₁' 10-500, vorzugsweise 19-100, besonders bevorzugt 20-30, beträgt.

8. Nachweissystem gemäß Anspruch 1, wobei die dritte Sonde die Struktur der Formel IV aufweist:
X₂'-T₂'-T₁'-P₁'-X₁' (IV)
wobei X₂' ein reverses Komplement des 5'-Ende-Universalamplifikationsprimers ist;
T₂' eine spezifische Sequenz am 5'-Ende eines Targeting-Gens ist;
T₁' eine spezifische Sequenz am 3'-Ende eines Targeting-Gens ist;
P₁' eine Strichcode-Indexsequenz ist, die die Gen-Amplifikationskombination markiert;
X₁' ein reverses Komplement des 3'-Ende-Universalamplifikationsprimers ist.

9. Verfahren zum quantitativen PCR-Nachweis, umfassend die Schritte:
(a) Bereitstellen einer nachzuweisenden Probe und des oberflächensondenbasierten quantitativen PCR-Nachweissystems gemäß Anspruch 1;
(b) in Gegenwart von Ligase und Polymerase unter Bedingungen, die für eine Ligationsreaktion und PCR-Amplifikation geeignet sind: Verwenden des quantitativen PCR-Nachweissystems zur Durchführung einer PCR-Amplifikation mit der nachzuweisenden Probe;
(c) Nachweisen des Signals des ersten nachweisbaren Markers des einen oder der mehreren flächenquantitativen Teilnachweisbereichs auf dem festen Träger während oder nach der PCR-Amplifikation; und
(d) Analysieren des nachgewiesenen Signals des ersten nachweisbaren Markers, wobei man ein quantitatives Nachweisergebnis der nachzuweisenden Probe erhält.

10. Kit zum quantitativen PCR-Nachweis, umfassend:
(a) einen ersten Behälter und einen Festphasenträger, der sich in dem ersten Behälter befindet;
wobei eine Primärfläche des Festphasenträgers mit n Teilnachweisbereichen versehen ist, wobei n eine positive ganze Zahl ≥ 2 ist und wenigstens ein Teilnachweisbereich ein flächenquantitativer Teilnachweisbereich ist;
wobei jeder der flächenquantitativen Teilnachweisbereiche unabhängig mit einer Quenchingprodukt-abfangenden Nucleinsäure immobilisiert ist, die Quenchingprodukt-abfangenden Nucleinsäure eine einzelsträngige Nucleinsäure ist und ein Ende der Quenchingprodukt-abfangenden Nucleinsäure auf der Oberfläche des Festphasenträgers immobilisiert ist und die Quenchingprodukt-abfangenden Nucleinsäure einen ersten nachweisbaren Marker aufweist, der aus der Gruppe ausgewählt ist, die aus einem Fluorophor, einem Chemilumineszenzmarker (Leuchtmarker) und einem Quantenpunkt besteht;
(b) einen zweiten Behälter und eine erste Sonde, eine zweite Sonde und eine Ligase in dem zweiten Behälter, wobei die Ligase die erste Sonde und die zweite Sonde zu einer dritten Sonde verbindet;
(c) einen dritten Behälter und ein Primerpaar, das sich in dem dritten Behälter befindet, wobei das Primerpaar ein Primerpaar zum Amplifizieren der dritten Sonde ist, wobei das Primerpaar einen ersten Primer und einen zweiten Primer umfasst, wobei der erste Primer und/oder der zweite Primer ein quenchender Primer ist und ein Ende oder eine Seite des quenchenden Primers mit einem Quencher verbunden ist,
außerdem das durch den quenchenden Primer durch Amplifizieren der dritten Sonde erzeugte quenchende Amplifikationsprodukt und wenigstens eine der Quenchingprodukt-abfangenden Nucleinsäuren des flächenquantitativen Teilnachweisbereichs unter Bildung einer doppelsträngigen Struktur miteinander kombiniert sein können und in der doppelsträngigen Struktur der Quencher des quenchenden Amplifikationsprodukts bewirkt, dass das Signal des ersten nachweisbaren Markers (wie eines Fluorophors) der abfangenden Nucleinsäure gelöscht wird;
(d) einen optionalen vierten Behälter und einen Puffer oder eine Pufferkomponente für die PCR-Amplifikation in dem vierten Behälter;
(e) einen optionalen fünften Behälter und eine Polymerase für die PCR-Amplifikation in dem fünften Behälter; und
(f) optionale Anleitungen, die Verfahren zum quantitativen PCR-Nachweis beschreiben;
wobei die erste Sonde die Struktur von Formel II aufweist:
X₂'-T₂' (II)
wobei X₂' ein reverses Komplement des 5'-Ende-Universalamplifikationsprimers ist;
T₂' ein reverses Komplement der spezifischen Sequenz am 5'-Ende eines Targeting-Gens ist:
wobei die zweite Sonde die Struktur von Formel III aufweist:
T₁'-P₁'-X₁' (III)
wobei T₁' ein reverses Komplement der spezifischen Sequenz am 3'-Ende eines Targeting-Gens ist;
P₁' eine Strichcode-Indexsequenz einer Marker-Gen-Amplifikationskombination ist;
X₁' ein reverses Komplement des 3'-Ende-Universalamplifikationsprimers ist.

## Revendications

1. Système de détection par PCR quantitative basé sur une sonde de surface, le système de détection comprenant :
(a) un support en phase solide, une surface primaire du support en phase solide étant pourvue de n sous-régions de détection, dans lequel n est un entier positif ≥ 2, et au moins une sous-région de détection étant une sous-région de détection de surface quantitative ;
dans lequel chacune des sous-régions de détection de surface quantitative est indépendamment immobilisée avec un acide nucléique de capture du produit d'extinction, l'acide nucléique de capture du produit d'extinction étant un acide nucléique simple brin, une extrémité de l'acide nucléique de capture du produit d'extinction étant immobilisée sur la surface du support en phase solide, et l'acide nucléique de capture du produit d'extinction présentant un premier marqueur détectable choisi dans le groupe constitué d'un fluorophore, d'un marqueur chimioluminescent, d'un point quantique, et d'une combinaison de ceux-ci ;
(b) une première sonde ;
(c) une deuxième sonde ;
(d) une ligase pour connecter la première sonde et la deuxième sonde afin de former la troisième sonde ;
(e) une paire d'amorces pour amplifier la troisième sonde, la paire d'amorces comprenant une première amorce et une deuxième amorce, au moins l'une de la première amorce et de la deuxième amorce étant une amorce d'extinction, une extrémité ou un côté de l'amorce d'extinction étant connecté à un agent d'extinction (quencher),
en outre, le produit d'amplification d'extinction généré par l'amorce d'extinction par amplification et au moins un des acides nucléiques de capture du produit d'extinction de la sous-région de détection de surface quantitative peuvent être combinés pour former une structure double brin, et dans la structure double brin, l'agent d'extinction du produit d'amplification d'extinction provoque l'extinction du signal du premier marqueur détectable (tel qu'un fluorophore) de l'acide nucléique de capture ;
dans lequel la première sonde a la structure de la Formule II :
X2'-T2' (II)
dans lequel, X2' est le complément inverse de l'amorce universelle d'amplification à l'extrémité 5' ;
T2' est le complément inverse de la séquence spécifique à l'extrémité 5' d'un gène cible ;
dans lequel la deuxième sonde a la structure de la Formule III :
T1'-P1'-X1' (III)
dans lequel, T1' est le complément inverse de la séquence spécifique à l'extrémité 3' d'un gène cible ;
P1' est une séquence d'index de code-barres d'une combinaison d'amplification du gène marqueur ;
X1' est le complément inverse de l'amorce universelle d'amplification à l'extrémité 3'.

2. Système de détection selon la revendication 1, dans lequel le rapport (Q1/Q0) du nombre Q1 de produits d'amplification d'extinction situés dans la distance d'hybridation effective par rapport à la sonde de surface au nombre Q0 de la sonde de surface correspondante (Q1/Q0) est de 0,5 à 100, de préférence de 1 à 10.

3. Système de détection selon la revendication 1, dans lequel la longueur (nt) de X2' est de 15 à 50, de préférence de 19 à 36, plus préférablement de 20 à 35.

4. Système de détection selon la revendication 1, dans lequel la longueur (nt) de T2' est de 15 à 60, de préférence de 19 à 36, plus préférablement de 20 à 35.

5. Système de détection selon la revendication 1, dans lequel l'acide nucléique de capture du produit d'extinction a la structure suivante de la Formule I :
B0-B1-B2-B3 (I)
dans lequel,
B0 est un groupe de liaison connectant l'acide nucléique de capture du produit d'extinction au support en phase solide ;
B1 est absent ou est une région de transition flexible choisie dans le groupe constitué de : un fragment d'acide nucléique de transition flexible d'une longueur de 1 à 100 nt, un fragment de polymère de transition flexible d'une longueur de 1 à 10 nm, et une combinaison de ceux-ci ;
B2 est une région de capture, dans laquelle la séquence de la région de capture est substantiellement complémentaire ou complètement complémentaire à la séquence de l'amorce d'extinction ;
B3 est absent ou est une région de séquence à l'extrémité 3' d'une longueur de 1 à 20 nt.

6. Système de détection selon la revendication 1, dans lequel la longueur (nt) de T1' est de 15 à 60, de préférence de 19 à 36, plus préférablement de 20 à 35.

7. Système de détection selon la revendication 1, dans lequel la longueur (nt) de P1' est de 10 à 500, de préférence de 19 à 100, plus préférablement de 20 à 30.

8. Système de détection selon la revendication 1, dans lequel la troisième sonde a la structure de la Formule IV :
X2'-T2'-T1'-P1'-X1' (IV)
dans lequel, X2' est le complément inverse de l'amorce universelle d'amplification à l'extrémité 5' ;
T2' est la séquence spécifique à l'extrémité 5' d'un gène cible ;
T1' est la séquence spécifique à l'extrémité 3' d'un gène cible ;
P1' est une séquence d'index de code-barres qui marque la combinaison d'amplification du gène ;
X1' est le complément inverse de l'amorce universelle d'amplification à l'extrémité 3'.

9. Procédé de détection par PCR quantitative, comprenant les étapes consistant à :
(a) fournir un échantillon à détecter et le système de détection par PCR quantitative basé sur une sonde de surface selon la revendication 1 ;
(b) en présence d'une ligase et d'une polymérase, dans des conditions appropriées à la réaction de ligation et à l'amplification par PCR, utiliser le système de détection par PCR quantitative pour effectuer une amplification par PCR sur l'échantillon à détecter ;
(c) détecter le signal du premier marqueur détectable d'une ou plusieurs sous-régions de détection de surface quantitative sur le support en phase solide pendant ou après l'amplification par PCR ; et
(d) analyser le signal détecté du premier marqueur détectable pour obtenir un résultat de détection quantitative de l'échantillon à détecter.

10. Kit de détection par PCR quantitative, comprenant :
(a) un premier récipient et un support en phase solide situé dans le premier récipient, une surface primaire du support en phase solide étant pourvue de n sous-régions de détection, dans lequel n est un entier positif ≥ 2, et au moins une sous-région de détection étant une sous-région de détection de surface quantitative ;
dans lequel chacune des sous-régions de détection de surface quantitative est indépendamment immobilisée avec un acide nucléique de capture du produit d'extinction, l'acide nucléique de capture du produit d'extinction étant un acide nucléique simple brin, une extrémité de l'acide nucléique de capture du produit d'extinction étant immobilisée sur la surface du support en phase solide, et l'acide nucléique de capture du produit d'extinction présentant un premier marqueur détectable choisi dans le groupe constitué d'un fluorophore, d'un marqueur chimioluminescent (marqueur luminescent), d'un point quantique ;
(b) un deuxième récipient et une première sonde, une deuxième sonde, et une ligase dans le deuxième récipient, la ligase liant la première sonde et la deuxième sonde en une troisième sonde ;
(c) un troisième récipient et une paire d'amorces situés dans le troisième récipient, la paire d'amorces étant une paire d'amorces pour amplifier la troisième sonde, la paire d'amorces comprenant une première amorce et une deuxième amorce, au moins l'une de la première amorce et de la deuxième amorce étant une amorce d'extinction, et une extrémité ou un côté de l'amorce d'extinction étant connecté à un agent d'extinction (quencher),
en outre, le produit d'amplification d'extinction généré par l'amorce d'extinction par amplification de la troisième sonde et au moins un des acides nucléiques de capture du produit d'extinction de la sous-région de détection de surface quantitative peuvent être combinés pour former une structure double brin, et dans la structure double brin, l'agent d'extinction du produit d'amplification d'extinction provoque l'extinction du signal du premier marqueur détectable (tel qu'un fluorophore) de l'acide nucléique de capture ;
(d) un quatrième récipient optionnel et un tampon ou un composant tampon pour l'amplification par PCR dans le quatrième récipient ;
(e) un cinquième récipient optionnel et une polymérase pour l'amplification par PCR dans le cinquième récipient ; et
(f) des instructions optionnelles décrivant les méthodes de détection par PCR quantitative ; dans lequel la première sonde a la structure de la Formule II :
X₂'-T₂' (II)
dans lequel, X2' est le complément inverse de l'amorce universelle d'amplification à l'extrémité 5' ;
T2' est le complément inverse de la séquence spécifique à l'extrémité 5' d'un gène cible ; dans lequel la deuxième sonde a la structure de la Formule III :
T1'-P1'-X1' (III)
dans lequel, T1' est le complément inverse de la séquence spécifique à l'extrémité 3' d'un gène cible ;
P1' est une séquence d'index de code-barres d'une combinaison d'amplification du gène marqueur ;
X1' est le complément inverse de l'amorce universelle d'amplification à l'extrémité 3'.
